# EUROPEAN PATENT APPLICATION

(11) **EP 4 578 396 A1**
(43) Date of publication of application: **02.07.2025**
(21) Application number: 23857008.9
(22) Date of filing: 07.07.2023
(51) Int. Cl.: A61B 6/00

(54) **INFORMATION PROCESSING DEVICE, INFORMATION PROCESSING METHOD, AND PROGRAM**

(30) Priority: 22.08.2022 JP 2022132090
(71) Applicant: FUJIFILM Corporation, Tokyo 106-8620 (JP)
(72) Inventor: MACHII, Yusuke, Ashigarakami-gun, Kanagawa 258-8538 (JP)
(74) Representative: Dehns Germany Partnerschaft mbB
(86) International application number: PCT/JP2023/025355
(87) International publication number: WO 2024/042891

(57) **Abstract**

An information processing apparatus according to the present disclosure includes: at least one processor, in which the processor is configured to: generate a difference image representing a difference between a low-energy image captured by irradiating a breast, in which a contrast agent is injected, with radiation having first energy and a high-energy image captured by irradiating the breast with radiation having second energy higher than the first energy; and determine an enhancement level of background mammary gland parenchyma of the breast based on the difference image and external information that is information other than the difference image.

## Description

### BACKGROUND OF THE INVENTION

### 1. Field of the Invention

The present disclosure relates to an information processing apparatus, an information processing method, and a program.

### 2. Description of the Related Art

Contrast-enhanced mammography, which acquires a low-energy image and a high-energy image by performing imaging by irradiating a breast in which a contrast agent is injected with radiation having different energies and obtains a difference between the low-energy image and the high-energy image to generate an image in which a lesion or the like is contrast-enhanced, is known. In recent years, since the contrast-enhanced mammography has been included in a comprehensive guideline for breast cancer image diagnosis called a breast imaging reporting and data system (BI-RADS), there is a high possibility that the contrast-enhanced mammography will be widely used as a standard diagnosis method.

However, it is difficult to perform the interpretation of the image obtained by the contrast-enhanced mammography. One of the reasons for the difficulty is an effect of background mammary gland parenchymal enhancement (BPE) due to the contrast agent. The BPE represents a level of enhancement of a normal structure of a mammary gland via the contrast agent, and the visibility of the enhanced lesion greatly varies depending on the level of the BPE. Therefore, in BI-RADS, it is recommended to evaluate and describe the enhancement level of background mammary gland parenchyma in a case of the interpretation. Hereinafter, the enhancement level of the background mammary gland parenchyma will be referred to as a "BPE level", and the enhancement region of the background mammary gland parenchyma will be referred to as a "BPE region". The BPE level represents a ratio of the enhanced mammary gland parenchyma to the background mammary gland.

As described above, since the difficulty of the interpretation is high in the contrast-enhanced mammography, it is desired to support even a doctor who is not accustomed to the interpretation so that standard interpretation can be performed. For example, WO2019/104252A discloses a technology of receiving an image of an inside of a breast and automatically classifying tissues by using a neural network. The classification of the tissues includes the classification of the mammary gland parenchyma.

### SUMMARY OF THE INVENTION

However, WO2019/104252A describes that the classification of the mammary gland parenchyma is performed, but does not disclose the determination of the BPE level. Therefore, in the technology described in WO2019/104252A, it is not possible to sufficiently support the interpretation in the contrast-enhanced mammography.

An object of the present disclosed technology is to provide an information processing apparatus, an information processing method, and a program capable of improving support for interpretation in contrast-enhanced mammography.

In order to achieve the above-described object, the present disclosure provides an information processing apparatus comprising: at least one processor, in which the processor is configured to: generate a difference image representing a difference between a low-energy image captured by irradiating a breast, in which a contrast agent is injected, with radiation having first energy and a high-energy image captured by irradiating the breast with radiation having second energy higher than the first energy; and determine an enhancement level of background mammary gland parenchyma of the breast based on the difference image and external information that is information other than the difference image.

It is preferable that the processor is configured to: extract a first feature value by inputting the difference image to a first machine learned model; and determine the enhancement level based on the first feature value and the external information.

It is preferable that the processor is configured to: determine the enhancement level by combining the external information with the first feature value and inputting the first feature value combined with the external information to a second machine learned model.

It is preferable that the second machine learned model classifies the enhancement level into a plurality of classes.

It is preferable that the second machine learned model specifies an enhancement region of the background mammary gland parenchyma via segmentation from the difference image and displays the specified enhancement region on the difference image, the low-energy image, or the high-energy image.

It is preferable that the processor is configured to: extract the first feature value by inputting the difference image and the low-energy image to the first machine learned model.

It is preferable that the processor is configured to: extract a first feature value by inputting the difference image to a first machine learned model; extract a second feature value by inputting the external information to a third machine learned model; and determine the enhancement level based on the first feature value and the second feature value.

It is preferable that the processor is configured to: determine the enhancement level by combining the first feature value and the second feature value and inputting the combined feature value to a second machine learned model.

It is preferable that the processor is configured to: combine the first feature value and the second feature value in a column direction, a row direction, or a channel direction.

It is preferable that the processor is configured to: determine the enhancement level by calculating a matrix product of the first feature value and the second feature value and inputting a feature value obtained by the calculation of the matrix product to a second machine learned model.

It is preferable that the external information includes at least any one of imaging-related information that is information related to imaging or person-under-examination-related information that is information related to a person under an examination.

It is preferable that the imaging-related information includes at least one of an elapsed time after contrast agent injection, an injection amount of the contrast agent, a tube voltage, a breast thickness, a mammary gland volume, or a breast type, and the person-under-examination-related information includes at least one of age, a menstrual cycle, a body weight, presence or absence of menopause, a heart rate, or a blood pressure.

The present disclosure provides an information processing method comprising: generating a difference image representing a difference between a low-energy image captured by irradiating a breast, in which a contrast agent is injected, with radiation having first energy and a high-energy image captured by irradiating the breast with radiation having second energy higher than the first energy; and determining an enhancement level of background mammary gland parenchyma of the breast based on the difference image and external information that is information other than the difference image.

The present disclosure provides a program causing a computer to execute a process comprising: generating a difference image representing a difference between a low-energy image captured by irradiating a breast, in which a contrast agent is injected, with radiation having first energy and a high-energy image captured by irradiating the breast with radiation having second energy higher than the first energy; and determining an enhancement level of background mammary gland parenchyma of the breast based on the difference image and external information that is information other than the difference image.

According to the present disclosed technology, it is possible to provide the information processing apparatus, the information processing method, and the program capable of improving the support for the interpretation in the contrast-enhanced mammography.

### BRIEF DESCRIPTION OF THE DRAWINGS

Fig. 1 is a diagram showing an example of an overall configuration of a radiation image capturing system.
Fig. 2 is a block diagram showing an example of a configuration of an information processing apparatus.
Fig. 3 is a block diagram showing an example of functions implemented by a control unit of the information processing apparatus.
Fig. 4 is a flowchart schematically showing a flow of contrast-enhanced imaging processing.
Fig. 5 is a flowchart schematically showing a flow of determination processing.
Fig. 6 is a diagram showing an example of external information.
Fig. 7 is a diagram schematically showing BPE level determination processing via a BPE level determination processing unit.
Fig. 8 is a diagram showing an example of a determination result of a BPE level.
Fig. 9 is a diagram conceptually showing an example of configurations of a first machine learned model and a second machine learned model.
Fig. 10 is a diagram schematically showing BPE level determination processing according to a first modification example.
Fig. 11 is a diagram schematically showing BPE level determination processing according to a second modification example.
Fig. 12 is a diagram schematically showing BPE level determination processing according to a third modification example.
Fig. 13 is a diagram conceptually showing combination processing of a first feature map and a second feature map.
Fig. 14 is a diagram conceptually showing a modification example of the combination processing.
Fig. 15 is a diagram conceptually showing another modification example of the combination processing.
Fig. 16 is a diagram schematically showing BPE level determination processing according to a fourth modification example.

### DESCRIPTION OF THE PREFERRED EMBODIMENTS

Hereinafter, an embodiment of the present disclosure will be described in detail with reference to the accompanying drawings.

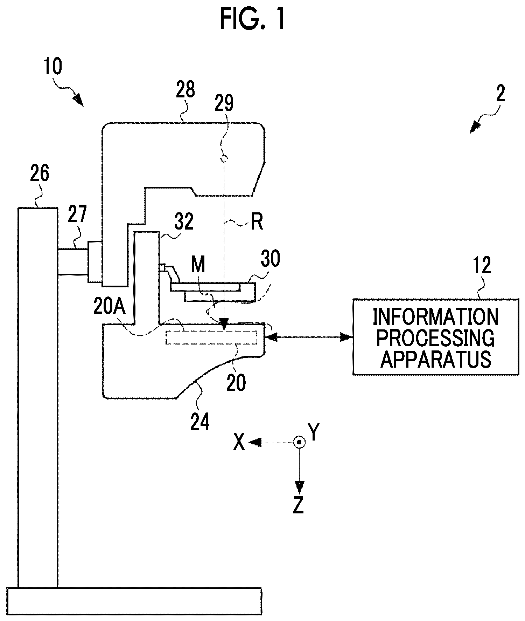

Fig. 1 shows an example of an overall configuration of a radiation image capturing system 2 according to the present embodiment. The radiation image capturing system 2 comprises a mammography apparatus 10 and an information processing apparatus 12. The information processing apparatus 12 is connected to a radiology information system (RIS), a picture archiving and communication system (PACS), and the like (none of which is shown) via a network or the like.

Fig. 1 shows an example of an appearance of the mammography apparatus 10. It should be noted that Fig. 1 shows an example of the appearance in a case in which the mammography apparatus 10 is seen from a left side of a person under an examination.

The mammography apparatus 10 is a radiography apparatus that operates under the control of the information processing apparatus 12 and that irradiates a breast M of a person under an examination, as a subject, with radiation R (for example, X-rays) from a radiation source 29 to capture a radiation image of the breast M.

As shown in Fig. 1, the mammography apparatus 10 comprises an imaging table 24, a base 26, an arm part 28, and a compression unit 32. A radiation detector 20 is disposed inside the imaging table 24. As shown in Fig. 1, in a case in which imaging is performed, in the mammography apparatus 10, the breast M of the person under an examination is positioned on the imaging table 24 by a user, such as a radiologist.

The radiation detector 20 detects the radiation R passing through the breast M as a subject. Specifically, the radiation detector 20 detects the radiation R passing through the breast M of the person under an examination, entering into the imaging table 24, and reaching a detection surface 20A of the radiation detector 20, and generates a radiation image based on the detected radiation R. The radiation detector 20 outputs image data representing the generated radiation image. Hereinafter, the series of operations of irradiating the breast with the radiation R from the radiation source 29 to generate the radiation image via the radiation detector 20 may be referred to as "imaging". The radiation detector 20 may be an indirect conversion type radiation detector that converts the radiation R into light beams and converts the converted light beams into charges, or may be a direct conversion type radiation detector that directly converts the radiation R into charges.

Hereinafter, two directions orthogonal to each other and parallel to the detection surface 20A will be referred to as an X direction and a Y direction. In addition, a direction orthogonal to the X direction and the Y direction will be referred to as a Z direction.

A compression plate 30 that is used for compressing the breast M in a case of performing the imaging is attached to the compression unit 32. The compression plate 30 is moved in a direction approaching or in a direction spaced away from the imaging table 24 by a compression plate drive unit (not shown) provided in the compression unit 32. The compression plate 30 is moved in a direction approaching the imaging table 24 to compress the breast M with the imaging table 24.

The arm part 28 can be rotated with respect to the base 26 by a shaft part 27. The shaft part 27 is fixed to the base 26, and the shaft part 27 and the arm part 28 are rotated integrally. Gears are provided in each of the shaft part 27 and the compression unit 32 of the imaging table 24, and the gears are switched between an engaged state and a non-engaged state, so that a state in which the compression unit 32 of the imaging table 24 and the shaft part 27 are connected to each other and are rotated integrally and a state in which the shaft part 27 is separated from the imaging table 24 and idles can be switched. The elements for switching between transmission and non-transmission of power of the shaft part 27 are not limited to the gears, and various mechanical elements can be used. The arm part 28 and the imaging table 24 can be separately rotated relative to the base 26 with the shaft part 27 as a rotation axis.

The mammography apparatus 10 can perform the imaging on each of the left and right breasts M from a plurality of directions by rotating the arm part 28. For example, it is possible to perform cranio-caudal (CC) imaging and medio-lateral oblique (MLO) imaging.

The radiation image capturing system 2 can perform "contrast-enhanced imaging" in which the imaging is performed in a state in which a contrast agent is injected in the breast M. Specifically, the radiation image capturing system 2 has a contrast enhanced digital mammography (CEDM) function of performing contrast enhancement via energy subtraction.

In the contrast-enhanced imaging, a low-energy image and a high-energy image are acquired by performing the imaging by irradiating the breast M, in which the contrast agent is injected, with the radiation R having different energies. In the present disclosure, a radiation image captured by the radiation R having a first energy will be referred to as a "low-energy image", and a radiation image captured by the radiation R having a second energy higher than the first energy will be referred to as a "high-energy image". Hereinafter, in a case in which the low-energy image and the high-energy image are not distinguished from each other, the low-energy image and the high-energy image will be simply referred to as a radiation image.

In the contrast-enhanced imaging, for example, an iodine contrast agent having a k absorption edge of 32 keV is used as the contrast agent. In the contrast-enhanced imaging in a case in which the iodine contrast agent is used, the first energy need only be set to be lower than the k absorption edge, and the second energy need only be set to be higher than the k absorption edge.

The contrast agent and the body tissue such as the mammary gland are different in absorption characteristics of the radiation R. Therefore, the high-energy image clearly shows the contrast agent in addition to the body tissue such as the mammary gland and the fat. On the other hand, in the low-energy image, the body tissue is clearly shown, but the contrast agent is hardly shown. Therefore, by taking a difference between the low-energy image and the high-energy image, it is possible to generate a difference image in which the mammary gland structure is erased and a lesion or the like stained with the contrast agent is enhanced. The lesion consists of, for example, new cells and is easily stained with the contrast agent.

The mammography apparatus 10 and the information processing apparatus 12 are connected by wired communication or wireless communication. The radiation image generated by the radiation detector 20 in the mammography apparatus 10 is output to the information processing apparatus 12 by wired communication or wireless communication via a communication interface (I/F) (not shown).

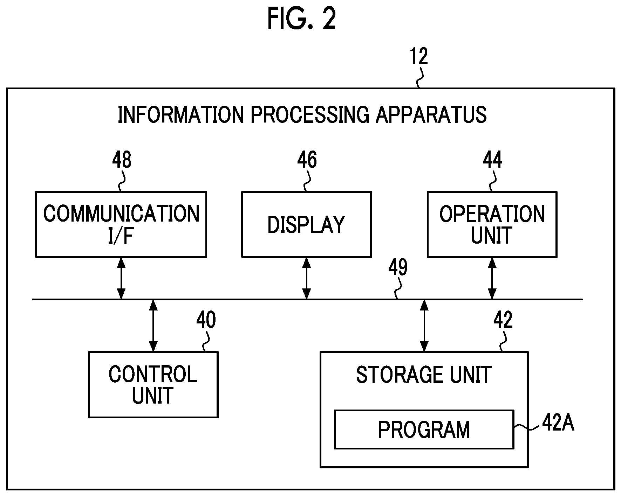

Fig. 2 shows an example of the configuration of the information processing apparatus 12. The information processing apparatus 12 comprises a control unit 40, a storage unit 42, an operation unit 44, a display 46, and a communication I/F 48. The control unit 40, the storage unit 42, the operation unit 44, the display 46, and the communication I/F 48 are connected to each other via a bus 49 such that various kinds of information can be exchanged.

The control unit 40 controls an overall operation of the radiation image capturing system 2. The control unit 40 is configured by, for example, a computer comprising a central processing unit (CPU), a read only memory (ROM), and a random access memory (RAM).

The storage unit 42 stores information related to radiography, the radiation image acquired from the mammography apparatus 10, and the like. In addition, the storage unit 42 stores a program 42A for the control unit 40 to perform various kinds of information processing described later and data for constructing various kinds of machine learned models described later. The storage unit 42 is, for example, a nonvolatile storage device such as a hard disk drive (HDD) or a solid state drive (SSD).

The operation unit 44 includes input devices such as various buttons, switches, a touch panel, a touch pen, and a mouse, which are operated by the user. The display 46 displays information related to the imaging, a radiation image obtained by the imaging, a determination result of BPE determination described later, and the like.

The communication I/F 48 performs communication of various kinds of data, such as information related to the radiography and the radiation image, with the mammography apparatus 10, the RIS, the PACS, and the like via wired communication or wireless communication.

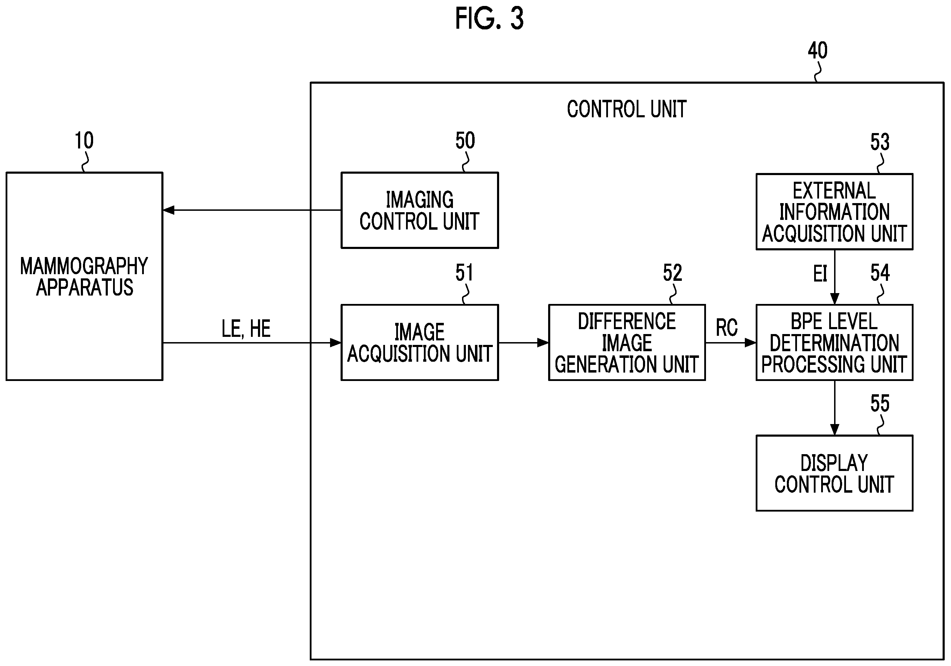

Fig. 3 shows an example of functions implemented by the control unit 40 of the information processing apparatus 12. The control unit 40 implements various functions by executing the processing based on the program 42A stored in the storage unit 42. The control unit 40 functions as an imaging control unit 50, an image acquisition unit 51, a difference image generation unit 52, an external information acquisition unit 53, a BPE level determination processing unit 54, and a display control unit 55.

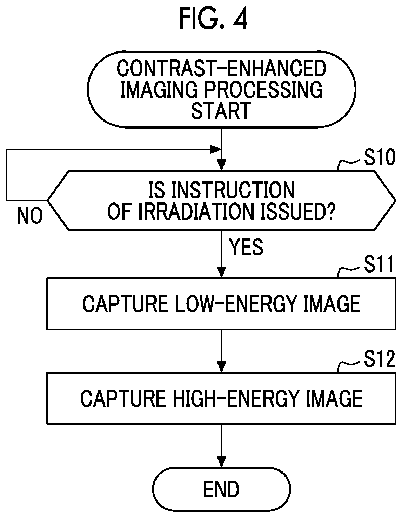

Fig. 4 schematically shows a flow of contrast-enhanced imaging processing. Processing via the imaging control unit 50 will be described with reference to Fig. 4.

First, before the imaging via the mammography apparatus 10 is started, the user, such as the radiologist, injects the contrast agent into the breast M of the person under an examination, positions the breast M in which the contrast agent is injected on the imaging table 24, and compresses the breast M with the compression plate 30.

In step S10, the imaging control unit 50 determines whether or not an instruction of the irradiation with the radiation R is received. In a case in which the instruction of the irradiation is received, the imaging control unit 50 outputs, in step S11, an instruction of the irradiation with the radiation R having the first energy to the mammography apparatus 10. In the mammography apparatus 10, a low-energy image LE is captured by emitting the first energy radiation R toward the breast M.

In next step S12, the imaging control unit 50 outputs an instruction of the irradiation with the radiation R having the second energy to the mammography apparatus 10. In the mammography apparatus 10, a high-energy image HE is captured by emitting the radiation R having the second energy toward the breast M. It should be noted that the high-energy image HE may be captured earlier than the low-energy image LE.

In a case in which the capturing of the low-energy image LE and the high-energy image HE of the breast M ends, the user releases the compression of the breast M for which the imaging ends.

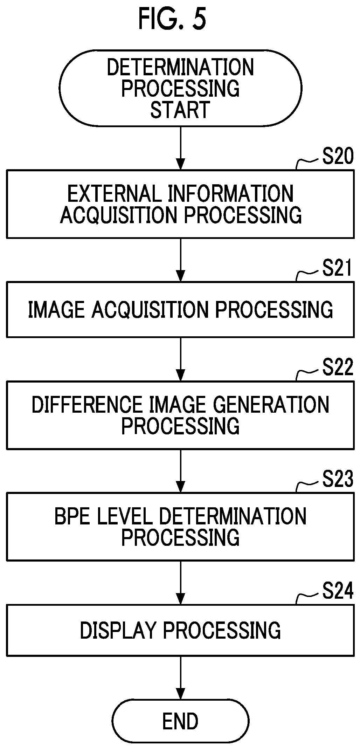

Fig. 5 schematically shows a flow of determination processing. Processing via the image acquisition unit 51, the difference image generation unit 52, the external information acquisition unit 53, the BPE level determination processing unit 54, and the display control unit 55 will be described with reference to Fig. 5.

In step S20, the external information acquisition unit 53 acquires external information EI, which is information other than a difference image RC described later. The external information EI includes imaging-related information that is information related to the imaging and person-under-examination-related information that is information related to the person under an examination. The external information acquisition unit 53 acquires information input from an external apparatus such as the RIS, information input by the user through the operation unit 44, and the like as the external information EI. It should be noted that the external information EI may be accumulated in the storage unit 42. In such a case, the external information acquisition unit 53 acquires the external information EI accumulated in the storage unit 42. In addition, the external information EI need only include at least one of the imaging-related information or the person-under-examination-related information.

In step S21, the image acquisition unit 51 acquires the low-energy image LE and the high-energy image HE captured by the above-described contrast-enhanced imaging processing.

In next step S22, the difference image generation unit 52 generates the difference image RC representing a difference between the low-energy image LE and the high-energy image HE. For example, the difference image generation unit 52 generates the difference image RC by subtracting an image obtained by multiplying the low-energy image LE by a predetermined coefficient from an image obtained by multiplying the high-energy image HE by a predetermined coefficient for each corresponding pixel.

In next step S23, the BPE level determination processing unit 54 performs BPE level determination processing, which will be described later, by using the difference image RC and the external information EI.

In next step S24, the display control unit 55 displays the determination result of the BPE level on the display 46. The display control unit 55 may display the difference image RC on the display 46 along with the determination result of the BPE level.

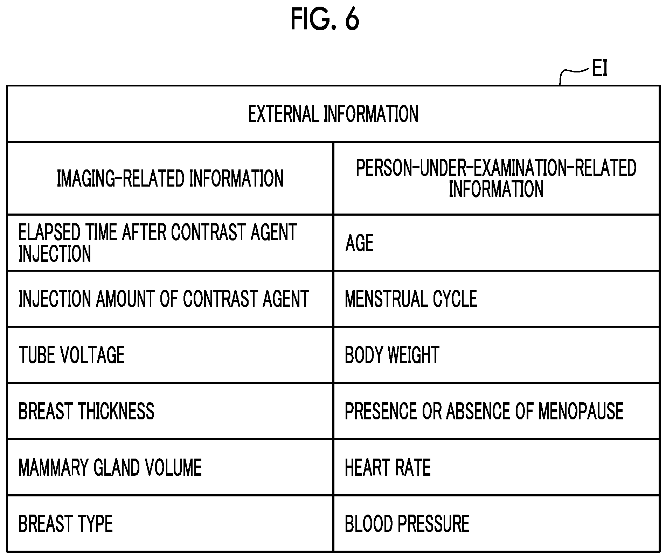

Fig. 6 shows an example of the external information EI. For example, the imaging-related information includes an elapsed time after the contrast agent injection, an injection amount of the contrast agent, a tube voltage, a breast thickness, a mammary gland volume, a breast type, and the like. For example, the person-under-examination-related information includes age, a menstrual cycle, a body weight, presence or absence of menopause, a heart rate, a blood pressure, and the like. These kinds of information are change factors caused by the change in the BPE level.

The imaging-related information need only include at least one of the elapsed time after the contrast agent injection, the injection amount of the contrast agent, the tube voltage, the breast thickness, the mammary gland volume, or the breast type. The person-under-examination-related information need only include at least one of the age, the menstrual cycle, the body weight, the presence or absence of menopause, the heart rate, or the blood pressure. It should be noted that it is preferable that each of the imaging-related information and the person-under-examination-related information includes a plurality of kinds of information.

The "elapsed time after the contrast agent injection" represents a time from the injection of the contrast agent into the breast M to the imaging. The "injection amount of the contrast agent" represents an amount of the contrast agent injected into the breast M. The "tube voltage" represents a voltage set in a radiation tube of the radiation source 29. The "breast thickness" represents a thickness of the breast M in a state of being compressed by the compression plate 30. The "mammary gland volume" represents a mammary gland tissue volume included in the breast M. The "breast type" represents a type of the breast M determined based on the mammary gland tissue volume. The breast type is classified into four types of high density, heterogeneous high density, scattered, and fatty in a descending order of the mammary gland tissue volume.

The "age" represents age of the person under an examination at the time of the imaging. The "menstrual cycle" represents a menstrual cycle of the person under an examination at the time of the imaging. The "body weight" represents a body weight of the person under an examination at the time of the imaging. The "presence or absence of menopause" represents presence or absence of menopause of the person under an examination at the time of the imaging. The "heart rate" represents a heart rate of the person under an examination at the time of the imaging. The "blood pressure" represents a blood pressure of the person under an examination at the time of the imaging.

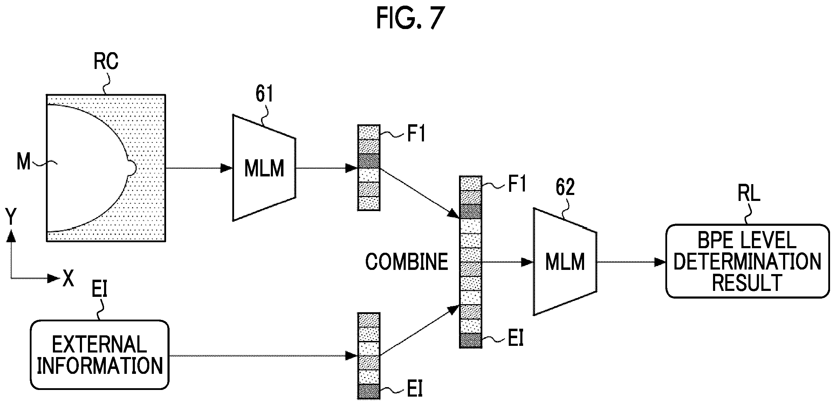

Fig. 7 schematically shows the BPE level determination processing via the BPE level determination processing unit 54. The BPE level determination processing unit 54 extracts a first feature map F1 from the difference image RC by inputting the difference image RC to a first machine learned model (MLM) 61 that functions as a feature value extraction unit. The first MLM 61 is configured by a convolutional neural network (CNN). It should be noted that the first feature map F1 is an example of a "first feature value" according to the present disclosed technology.

Next, the BPE level determination processing unit 54 determines the BPE level of the breast M by combining the external information EI with the first feature map F1 and inputting the first feature map F1 combined with the external information EI to a second MLM 62 that functions as a BPE level determination unit. The second MLM 62 outputs a determination result RL of the BPE level. The second MLM 62 is configured by a CNN, similarly to the first MLM 61. It should be noted that the external information EI is represented by a numerical value and is combined with the first feature map F1 as numerical data.

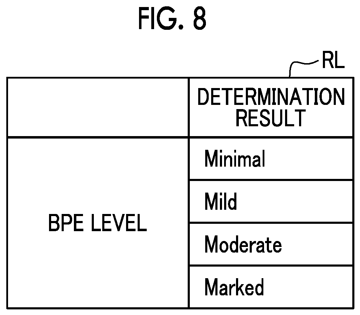

Fig. 8 shows an example of the determination result RL of the BPE level. As shown in Fig. 8, the determination results of the BPE level are classified into four classes of "Minimal", "Mild", "Moderate", and "Marked". A ratio of the contrast-enhanced mammary gland parenchyma to the background mammary gland is "Minimal" in a case of being less than 25%, "Mild" in a case of being 25% or more and less than 50%, "Moderate" in a case of being 50% or more and less than 75%, and "Marked" in a case of being 75% or more. In "Marked", there is a possibility that the lesion is buried in the background mammary gland and the lesion is invisible.

The second MLM 62 outputs, as the determination result RL, a class to which the BPE level of the breast M belongs among the four classes.

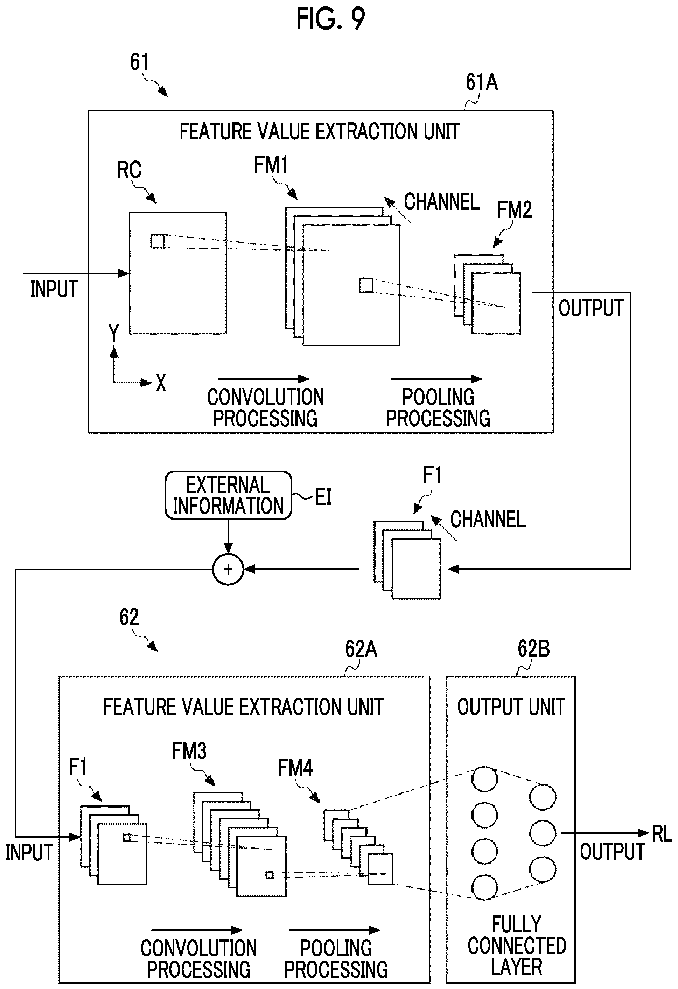

Fig. 9 conceptually shows an example of the configurations of the first MLM 61 and the second MLM 62. The first MLM 61 includes a feature value extraction unit 61A including a convolutional layer and a pooling layer.

The second MLM 62 includes a feature value extraction unit 62A and an output unit 62B. The feature value extraction unit 62A is an intermediate layer including a convolutional layer and a pooling layer. The output unit 62B is an output layer including a fully connected layer.

The difference image RC is input to the feature value extraction unit 61A of the first MLM 61. The feature value extraction unit 61A extracts a feature value by executing convolution processing and pooling processing on the input difference image RC. In the example shown in Fig. 9, the feature value extraction unit 61A generates a three-channel feature map FM1 by executing the convolution processing on the difference image RC, and generates a three-channel feature map FM2 having a reduced size by executing the pooling processing on the generated feature map FM1. In the present embodiment, the feature value extraction unit 61A outputs the three-channel feature map FM2 as the first feature map F1. The number of channels is determined by the number of filters used in a case in which the convolution processing is performed.

The first feature map F1 combined with the external information EI is input to the feature value extraction unit 62A of the second MLM 62. The feature value extraction unit 61A extracts a feature value by executing the convolution processing and the pooling processing on the input first feature map F1. In the example shown in Fig. 9, the feature value extraction unit 62A generates a six-channel feature map FM3 by executing the convolution processing on the first feature map F1 combined with the external information EI, and generates a six-channel feature map FM4 having a reduced size by executing the pooling processing on the generated feature map FM3.

The output unit 62B performs the class classification based on the feature value extracted by the feature value extraction unit 62A, and outputs a result of the class classification as the determination result RL. In the example shown in Fig. 9, the feature map FM4 is input from the feature value extraction unit 62A to the output unit 62B. The output unit 62B performs the class classification based on the feature map FM4.

The number of convolutional layers, the number of pooling layers, the number of filters, and the like included in each of the first MLM 61 and the second MLM 62 can be changed as appropriate.

For example, the first MLM 61 and the second MLM 62 have been trained through machine learning as one neural network as a whole. In a training phase, the first MLM 61 and the second MLM 62 are generated by training the learning model through machine learning using the known difference image RC, the external information EI, and ground-truth data of the BPE level as training data. The learning model is trained through machine learning using the external information EI that is a change factor caused by the change in the BPE level, so that the determination accuracy of the BPE level is improved as compared with a case in which only the difference image RC is used.

As described above, in the present embodiment, the BPE level is determined by using the external information EI that is the change factor caused by the change in the BPE level, in addition to the feature value extracted from the difference image RC, and thus the BPE level can be determined with high accuracy. As a result, the doctor can easily and accurately determine the BPE level, and the support for the interpretation in the contrast-enhanced mammography is improved.

Hereinafter, various modification examples of the above-described embodiment will be described.

### [First Modification Example]

The first modification example is different from the above-described embodiment only in the BPE level determination processing via the BPE level determination processing unit 54. In the present modification example, the BPE level determination processing unit 54 detects the BPE region from the difference image RC by performing segmentation.

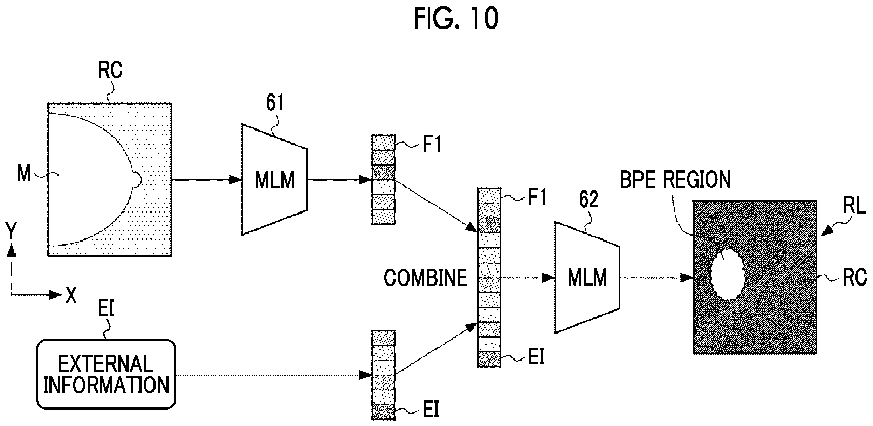

Fig. 10 schematically shows the BPE level determination processing according to the first modification example. In the present modification example, for example, the second MLM 62 is configured by a U-net, which is one kind of a CNN, and performs the class classification of each pixel of the image for the BPE level.

The second MLM 62 specifies the BPE region corresponding to a predetermined BPE level, and displays the BPE region on the difference image RC. In the present modification example, the second MLM 62 outputs the difference image RC in which the BPE region is specified, as the determination result RL of the BPE level. It should be noted that the second MLM 62 may display the specified BPE region on the low-energy image LE or the high-energy image HE and output the low-energy image LE or high-energy image HE on which the specified BPE region is displayed, as the determination result RL of the BPE level.

The BPE region shown in Fig. 10 is, for example, the region classified as "Marked". In the BPE level determination processing according to the first modification example, processing other than the BPE level determination processing is the same as in the BPE level determination processing according to the above-described embodiment.

In the example shown in Fig. 10, two regions of the BPE region and the other region are displayed in a distinguished manner, but a plurality of BPE regions may be displayed in accordance with the BPE level. For example, regions corresponding to the BPE levels of "Minimal", "Mild", "Moderate", and "Marked" may be displayed in a distinguished manner.

In addition, the second MLM 62 may detect the BPE region via object detection and display a rectangular bounding box including the BPE region on the difference image RC, the low-energy image LE, or the high-energy image HE. In this case, the second MLM 62 is configured by, for example, an R-CNN (regions with CNN features), which is one kind of a CNN.

### [Second Modification Example]

The second modification example is different from the above-described embodiment in that the BPE level determination processing is performed by using the low-energy image LE acquired by the contrast-enhanced imaging processing in addition to the difference image RC.

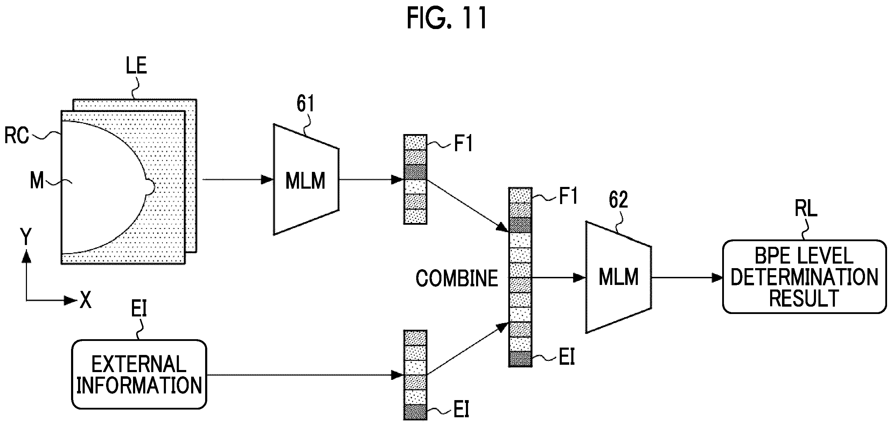

Fig. 11 schematically shows the BPE level determination processing according to the second modification example. In the present modification example, the BPE level determination processing unit 54 inputs the difference image RC and the low-energy image LE to the first MLM 61. For example, the BPE level determination processing unit 54 combines the difference image RC and the low-energy image LE in a channel direction and inputs the combined image to the first MLM 61.

In the present modification example as well, as in the above-described embodiment, the BPE level determination processing unit 54 determines the BPE level of the breast M by combining the external information EI with the first feature map F1 output from the first MLM 61 and inputting the first feature map F1 combined with the external information EI to the second MLM 62.

The mammary gland structure is clearly shown in the low-energy image LE. Therefore, by performing the BPE level determination processing by using the low-energy image LE in addition to the difference image RC and the external information EI, the BPE level can be determined with higher accuracy.

It should be noted that the BPE level determination processing may be performed by using the high-energy image HE in addition to the difference image RC and the external information EI. In addition, the BPE level determination processing may be performed by using the low-energy image LE and the high-energy image HE in addition to the difference image RC and the external information EI.

### [Third Modification Example]

The third modification example is different from the above-described embodiment in that the determination of the BPE level is performed by combining the feature value extracted from the external information EI with the feature value extracted from the difference image RC.

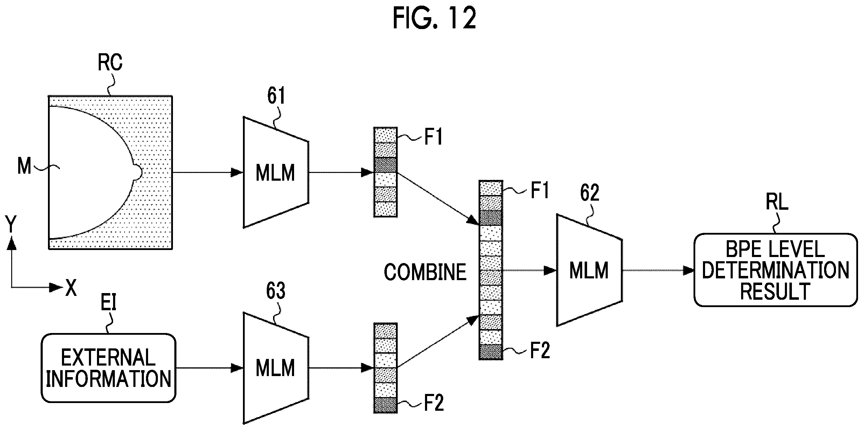

Fig. 12 schematically shows the BPE level determination processing according to the third modification example. In the present modification example, the BPE level determination processing unit 54 extracts a second feature map F2 from the external information EI by inputting the external information EI to a third MLM 63 that functions as a feature value extraction unit. The third MLM 63 has the same configuration as the first MLM 61, and is configured by a CNN. It should be noted that the second feature map F2 is an example of a "second feature value" according to the present disclosed technology.

In the present modification example, the BPE level determination processing unit 54 combines the first feature map F1 output from the first MLM 61 and the second feature map F2 output from the third MLM 63, and inputs the combined feature map to the second MLM 62. The second MLM 62 performs the extraction of the feature value and the class classification based on the combined first feature map F1 and second feature map F2, to output the determination result RL of the BPE level.

In the present modification example, for example, the first MLM 61, the second MLM 62, and the third MLM 63 have been trained through machine learning as one neural network as a whole. In the training phase, the first MLM 61, the second MLM 62, and the third MLM 63 are generated by training the learning model through machine learning using the known difference image RC, the known external information EI, and the ground-truth data of the BPE level as training data.

In the present modification example, since the determination of the BPE level is performed by combining the feature value extracted from the external information EI with the feature value extracted from the difference image RC, the robustness against the change in the external information EI is improved.

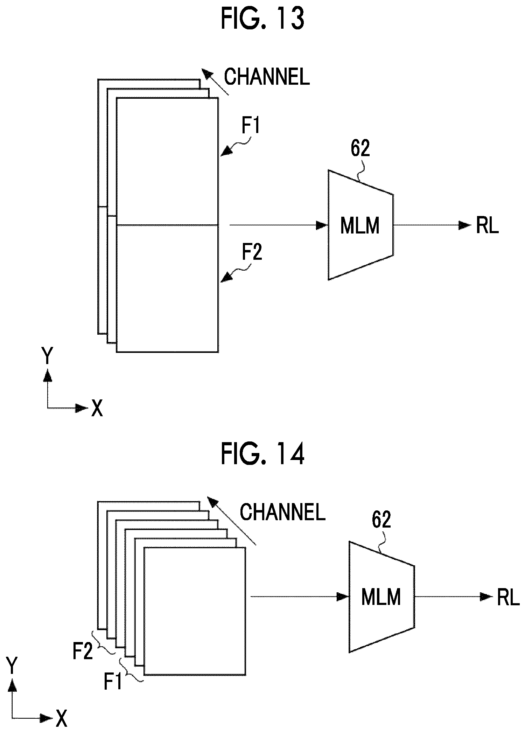

Fig. 13 conceptually shows combination processing of the first feature map F1 and the second feature map F2. The BPE level determination processing unit 54 combines the first feature map F1 with the second feature map F2 for each channel. In the example shown in Fig. 13, the first feature map F1 and the second feature map F2 are combined in a column direction (Y direction). That is, in the present modification example, an overall size of the combined first feature map F1 and second feature map F2 is increased. It should be noted that the first feature map F1 and the second feature map F2 may be combined in a row direction (X direction).

Various modifications can be made on the combination processing of combining the first feature map F1 and the second feature map F2.

Fig. 14 conceptually shows a modification example of the combination processing. In the present modification example, the BPE level determination processing unit 54 combines the first feature map F1 and the second feature map F2 in a superimposed manner in the channel direction. That is, in the present modification example, the total number of channels in the combined first feature map F1 and second feature map F2 increases.

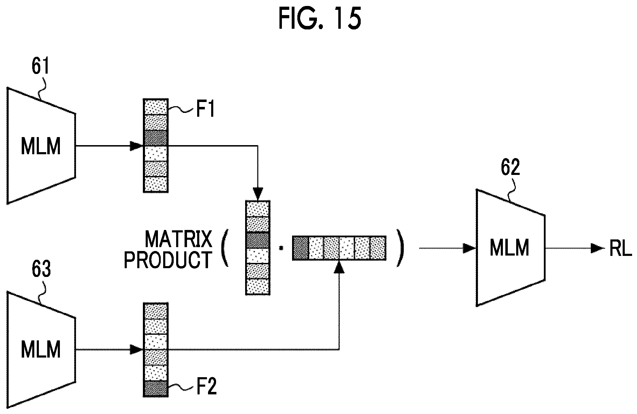

Fig. 15 conceptually shows another modification example of the combination processing. In the present modification example, the BPE level determination processing unit 54 calculates a matrix product of the first feature map F1 and the second feature map F2, and inputs the feature map obtained by the calculation of the matrix product to the second MLM 62.

It should be noted that the combination processing of combining the first feature map F1 and the second feature map F2 is not limited to the processing of combining the feature maps in the column direction (Y direction) or the row direction (X direction), the processing of combining the feature maps in the channel direction, and the processing of calculating the matrix product, and may be processing of calculating a corresponding element product, processing of calculating a corresponding element sum, or the like.

### [Fourth Modification Example]

The fourth modification example is different from the above-described embodiment in that the determination of the BPE level is performed by combining the external information EI is combined with the difference image RC.

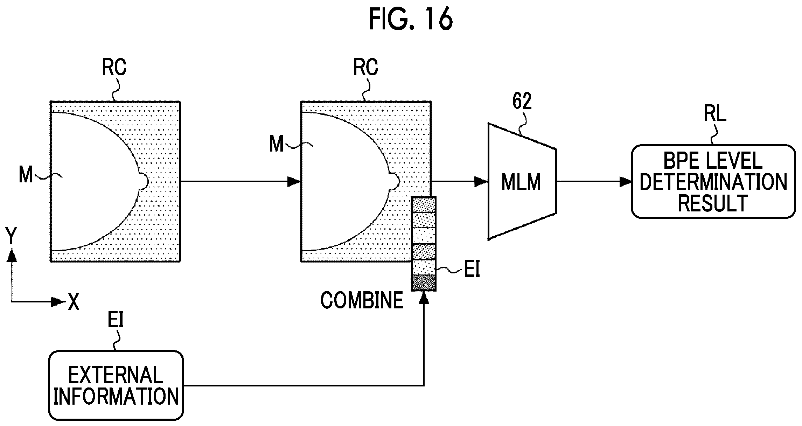

Fig. 16 schematically shows the BPE level determination processing according to the fourth modification example. In the present modification example, the BPE level determination processing unit 54 combines the external information EI with the difference image RC instead of the feature value of the difference image RC, and inputs the difference image RC combined with the external information EI to the second MLM 62. The second MLM 62 outputs the determination result RL of the BPE level by performing the extraction of the feature value and the class classification based on the difference image RC combined with the external information EI.

### [Other Modification Examples]

In the above-described embodiment, the determination of the BPE level is performed based on the difference image RC and the external information EI, but the determination of the BPE level may be performed based on the difference image RC and the external information EI in addition to the mammary gland information extracted from the low-energy image LE. The mammary gland information is, for example, a mammary gland region image representing a region including the mammary gland structure. The mammary gland information is not limited to the mammary gland region image, and may be a mammary gland volume image representing the mammary gland volume for each pixel. The mammary gland information may be extracted from the high-energy image HE instead of the low-energy image LE. Further, the mammary gland information may be extracted from each of the low-energy image LE and the high-energy image HE.

In addition, in the above-described embodiment and respective modification examples, the first MLM 61, the second MLM 62, and the third MLM 63 are each configured by a CNN, but the present disclosure is not limited to the CNN, and need only be configured by a neural network capable of extracting the feature value. That is, each of the first MLM 61, the second MLM 62, and the third MLM 63 need only be configured by machine learning-based neural networks, such as a CNN and a multilayer perceptron (MLP) neural network.

In addition, in the above-described embodiment and respective modification examples, the second MLM 62 classifies the BPE level into the four classes and outputs the classified BPE level, but the number of classes to be classified is not limited to four and can be appropriately changed. Further, the second MLM 62 may be configured to output a numerical value representing the BPE level.

In addition, in the above-described embodiment and respective modification examples, as a hardware structure of a processing unit that executes various kinds of processing, such as the imaging control unit 50, the image acquisition unit 51, the difference image generation unit 52, the external information acquisition unit 53, the BPE level determination processing unit 54, and the display control unit 55, various processors shown below can be used.

The various processors include a graphics processing unit (GPU) as well as a CPU. Further, the various processors include, in addition to a general-purpose processor which executes software (program) and functions as various processing units, such as a CPU, a programmable logic device (PLD) that is a processor whose circuit configuration can be changed after manufacture, such as a field programmable gate array (FPGA), and a dedicated electrical circuit that is a processor having a circuit configuration which is designed for exclusive use in order to execute specific processing, such as an application-specific integrated circuit (ASIC).

One processing unit may be configured by one of the various processors or may be configured by combining two or more processors of the same type or different types (for example, by combining a plurality of FPGAs or combining a CPU and an FPGA). Further, a plurality of the processing units may be configured by one processor.

A first example of the configuration in which the plurality of processing units are configured by one processor is a form in which one processor is configured by combining one or more CPUs and the software and this processor functions as the plurality of processing units, as represented by computers such as a client and a server. A second example is a form of using a processor that implements the function of the entire system including the plurality of processing units via one integrated circuit (IC) chip, as represented by a system on a chip (SoC) or the like. In this way, as the hardware structure, the various processing units are configured by using one or more of the various processors described above.

Further, the hardware structure of the various processors is, more specifically, an electric circuit (circuitry) in which circuit elements such as semiconductor elements are combined.

In addition, in the above-described embodiment and respective modification examples, the aspect has been described in which the program 42A is stored in the storage unit 42 in advance, but the present disclosure is not limited to this. The program 42A may be provided in a form of being recorded in a non-transitory recording medium such as a compact disc read only memory (CD-ROM), a digital versatile disc read only memory (DVD-ROM), or a universal serial bus (USB) memory. Further, the program 42A may be downloaded from an external apparatus via a network.

The above-described embodiment and respective modification examples can be combined as appropriate as long as there is no contradiction.

The above-described contents and the above-shown contents are detailed descriptions of portions related to the present disclosed technology and are merely examples of the present disclosed technology. For example, the description of the configuration, the function, the operation, and the effect are the description of examples of the configuration, the function, the operation, and the effect of the parts according to the present disclosed technology. Therefore, it goes without saying that unnecessary parts may be deleted, new elements may be added, or replacements may be made with respect to the above-described contents and the above-shown contents within a range that does not deviate from the gist of the present disclosed technology. Further, the description of, for example, common technical knowledge that does not need to be particularly described to enable the implementation of the present disclosed technology is omitted in the above-described contents and the above-shown contents in order to avoid confusion and to facilitate the understanding of the portions related to the present disclosed technology.

All of the documents, the patent applications, and the technical standards described in the present specification are incorporated into the present specification by reference to the same extent as in a case in which the individual documents, patent applications, and technical standards are specifically and individually stated to be described by reference.

The following technology can be understood from the above description.

### [Supplementary Note 1]

An information processing apparatus comprising: at least one processor, in which the processor is configured to: generate a difference image representing a difference between a low-energy image captured by irradiating a breast, in which a contrast agent is injected, with radiation having first energy and a high-energy image captured by irradiating the breast with radiation having second energy higher than the first energy; and determine an enhancement level of background mammary gland parenchyma of the breast based on the difference image and external information that is information other than the difference image.

### [Supplementary Note 2]

The information processing apparatus according to supplementary note 1, in which the processor is configured to: extract a first feature value by inputting the difference image to a first machine learned model; and determine the enhancement level based on the first feature value and the external information.

### [Supplementary Note 3]

The information processing apparatus according to supplementary note 2, in which the processor is configured to: determine the enhancement level by combining the external information with the first feature value and inputting the first feature value combined with the external information to a second machine learned model.

### [Supplementary Note 4]

The information processing apparatus according to supplementary note 3, in which the second machine learned model classifies the enhancement level into a plurality of classes.

### [Supplementary Note 5]

The information processing apparatus according to supplementary note 3, in which the second machine learned model specifies an enhancement region of the background mammary gland parenchyma via segmentation from the difference image and displays the specified enhancement region on the difference image, the low-energy image, or the high-energy image.

### [Supplementary Note 6]

The information processing apparatus according to any one of supplementary notes 2 to 5, in which the processor is configured to: extract the first feature value by inputting the difference image and the low-energy image to the first machine learned model.

### [Supplementary Note 7]

The information processing apparatus according to supplementary note 1, in which the processor is configured to: extract a first feature value by inputting the difference image to a first machine learned model; extract a second feature value by inputting the external information to a third machine learned model; and determine the enhancement level based on the first feature value and the second feature value.

### [Supplementary Note 8]

The information processing apparatus according to supplementary note 7, in which the processor is configured to: determine the enhancement level by combining the first feature value and the second feature value and inputting the combined feature value to a second machine learned model.

### [Supplementary Note 9]

The information processing apparatus according to supplementary note 8, in which the processor is configured to: combine the first feature value and the second feature value in a column direction, a row direction, or a channel direction.

### [Supplementary Note 10]

The information processing apparatus according to supplementary note 7, in which the processor is configured to: determine the enhancement level by calculating a matrix product of the first feature value and the second feature value and inputting a feature value obtained by the calculation of the matrix product to a second machine learned model.

### [Supplementary Note 11]

The information processing apparatus according to any one of supplementary notes 1 to 10, in which the external information includes at least any one of imaging-related information that is information related to imaging or person-under-examination-related information that is information related to a person under an examination.

### [Supplementary Note 12]

The information processing apparatus according to supplementary note 11, in which the imaging-related information includes at least one of an elapsed time after contrast agent injection, an injection amount of the contrast agent, a tube voltage, a breast thickness, a mammary gland volume, or a breast type, and the person-under-examination-related information includes at least one of age, a menstrual cycle, a body weight, presence or absence of menopause, a heart rate, or a blood pressure.

## Claims

1. An information processing apparatus comprising:
at least one processor,
wherein the processor is configured to:
generate a difference image representing a difference between a low-energy image captured by irradiating a breast, in which a contrast agent is injected, with radiation having first energy and a high-energy image captured by irradiating the breast with radiation having second energy higher than the first energy; and
determine an enhancement level of background mammary gland parenchyma of the breast based on the difference image and external information that is information other than the difference image.

2. The information processing apparatus according to claim 1,
wherein the processor is configured to:
extract a first feature value by inputting the difference image to a first machine learned model; and
determine the enhancement level based on the first feature value and the external information.

3. The information processing apparatus according to claim 2,
wherein the processor is configured to:
determine the enhancement level by combining the external information with the first feature value and inputting the first feature value combined with the external information to a second machine learned model.

4. The information processing apparatus according to claim 3,
wherein the second machine learned model classifies the enhancement level into a plurality of classes.

5. The information processing apparatus according to claim 3,
wherein the second machine learned model specifies an enhancement region of the background mammary gland parenchyma via segmentation from the difference image and displays the specified enhancement region on the difference image, the low-energy image, or the high-energy image.

6. The information processing apparatus according to claim 2,
wherein the processor is configured to:
extract the first feature value by inputting the difference image and the low-energy image to the first machine learned model.

7. The information processing apparatus according to claim 1,
wherein the processor is configured to:
extract a first feature value by inputting the difference image to a first machine learned model;
extract a second feature value by inputting the external information to a third machine learned model; and
determine the enhancement level based on the first feature value and the second feature value.

8. The information processing apparatus according to claim 7,
wherein the processor is configured to:
determine the enhancement level by combining the first feature value and the second feature value and inputting the combined feature value to a second machine learned model.

9. The information processing apparatus according to claim 8,
wherein the processor is configured to:
combine the first feature value and the second feature value in a column direction, a row direction, or a channel direction.

10. The information processing apparatus according to claim 7,
wherein the processor is configured to:
determine the enhancement level by calculating a matrix product of the first feature value and the second feature value and inputting a feature value obtained by the calculation of the matrix product to a second machine learned model.

11. The information processing apparatus according to claim 1,
wherein the external information includes at least any one of imaging-related information that is information related to imaging or person-under-examination-related information that is information related to a person under an examination.

12. The information processing apparatus according to claim 11,
wherein the imaging-related information includes at least one of an elapsed time after contrast agent injection, an injection amount of the contrast agent, a tube voltage, a breast thickness, a mammary gland volume, or a breast type, and
the person-under-examination-related information includes at least one of age, a menstrual cycle, a body weight, presence or absence of menopause, a heart rate, or a blood pressure.

13. An information processing method comprising:
generating a difference image representing a difference between a low-energy image captured by irradiating a breast, in which a contrast agent is injected, with radiation having first energy and a high-energy image captured by irradiating the breast with radiation having second energy higher than the first energy; and
determining an enhancement level of background mammary gland parenchyma of the breast based on the difference image and external information that is information other than the difference image.

14. A program causing a computer to execute a process comprising:
generating a difference image representing a difference between a low-energy image captured by irradiating a breast, in which a contrast agent is injected, with radiation having first energy and a high-energy image captured by irradiating the breast with radiation having second energy higher than the first energy; and
determining an enhancement level of background mammary gland parenchyma of the breast based on the difference image and external information that is information other than the difference image.
